# EUROPEAN PATENT SPECIFICATION

(11) **EP 3 986 491 B1**
(45) Date of publication and mention of the grant of the patent: **23.08.2023**
(21) Application number: 20733293.3
(22) Date of filing: 19.06.2020
(51) Int. Cl.: A61L 27/52, A61L 27/50, A61L 27/38, B33Y 70/00

(54) **CROSSLINKABLE HYDROGEL COMPOSITIONS**
VERNETZBARE HYDROGELZUSAMMENSETZUNGEN
COMPOSITIONS D'HYDROGEL RÉTICULABLE

(30) Priority: 20.06.2019 EP 19181458
(43) Date of publication of application: 27.04.2022
(73) Proprietor: ETH Zürich, 8092 Zürich (CH)
(72) Inventor: TIBBITT, Mark, 8004 Zürich (CH); GUZZI, Elia, 8004 Zürich (CH)
(74) Representative: Schmitz, Joseph
(86) International application number: PCT/EP2020/067187
(87) International publication number: WO 2020/254626

(56) References cited:
- US-A1- 2017 319 506

## Description

### TECHNICAL FIELD

The present invention relates to cross-linkable hydrogels and their use as inks in direct ink writing (DIW) or their use as injectable vehicles, which hydrogels exhibit shear-thinning as well as self-healing.

### PRIOR ART

A hydrogel is a network of polymer chains that are generally hydrophilic in which water forms the phase in which the polymer chains are dispersed. Examples include plant or animal biopolymers such as collagen, alginate, hyaluronic acid or synthetic polymers such as poly(ethylene glycol) diacrylate (PEGDA).

Hydrogels may be useful in several applications and in particular can be useful in biological, biomedical or pharmaceutical applications where they may serve as either scaffolds for tissue engineering, wound repair materials, vehicles in injectable sustained release formulations or as biological models.

In said applications, the hydrogels are generally brought into a certain shape via several techniques, such as for example additive manufacturing (AM), which enables freeform and accurate deposition of the hydrogels, enabling on-demand fabrication of objects. Alternatively, cells- or drug-containing hydrogels may be injected directly into a corporeal site of medical application to form, for example, a localized depot body through which cells can migrate out- or towards it, or a drug is controllably released.

Efficient fabrication of biomaterial objects via AM requires suitable inks. Such inks need to satisfy specific physical constraints, defined by the AM technology, related to the material viscosity, flowability, self-healing rate, and gelation kinetics. The target application often introduces additional design parameters. For example, in tissue engineering the ink should be compatible with living cells while in drug delivery the ink should enable the controlled release of molecular therapeutics. To date, a range of inks tailored to a specific AM technology and/or application exist, enabling to provide constructs that can be used in tissue repair, for ex vivo drug screening and disease modeling, or for the design of patient-specific drug delivery systems (DDS).

Direct ink writing (DIW) is one of the main AM technologies used in particular for bio-fabrication applications. DIW operates by extruding a material ink via piston-, pneumatic-, or screw-driven robotic dispensing in defined locations to fabricate the final 3D construct. In general, inks suitable for DIW must demonstrate shear-induced flow during extrusion (i.e. shear-thinning) and rapid material reformation for shape retention following deposition (i.e. self-healing). On the other hand, shear-induced flow and shape retention immediately after deposition are also properties that are desirable in injectable formulations that may be used for sustained drug release and delivery.

While effectively bringing the hydrogels into a certain form is important, long-term shape retention and/or structural integrity of the hydrogel structure in the environment of intended use such as aqueous environments and/or biological environments is equally important.

US2017/0319506 A1 discloses shear thinning and self-healing polymer nanoparticle (PNP) gels comprising one or more gel-forming polymer and a nanoparticle. The gels can be used in biomedical application such as drug delivery systems for *in vitro* or *in vivo* study.

However, when the gels of US2017/0319506 A1 are exposed to an aqueous environment such as water or buffer solutions, they quickly erode and disintegrate, thereby limiting their use in aqueous or biological environments and in particular in long-term drug screening and disease modeling or in depot formulations.

It is therefore desirable to provide inks which are suitable for DIW in the sense that they should exhibit shear-thinning, self-healing and which moreover allow for the manufacture of constructs having extended structural stability in aqueous environments and thus may render possible their use both in long-term drug screening and disease modeling or in extended release formulations, in *in vitro* and *in vivo* applications.

### SUMMARY OF THE INVENTION

The present applications therefore provides a cross-linkable hydrogel composition as defined in claim 1 that in addition to exhibiting shear thinning and self-healing also exhibits dimensional stability in aqueous and/or biological environments. The hydrogels according to the present invention are furthermore unexpectedly robust in the sense that they can be accommodate a variety of cross-linkable hydrogel precursors comprised in the cross-linkable hydrogel composition of the present invention.

It is an object of the present invention to provide a cross-linkable hydrogel composition comprising an aqueous base carrier composition and a cross-linkable hydrogel precursor, wherein the aqueous base carrier composition comprises one or more polymers and one or more nanoparticles, wherein the one or more polymers are selectively adsorbed to the one or more nanoparticles, and wherein the one or more polymers and the one or more nanoparticles form a transient shear-thinning and self-healing hydrogel and wherein the one or more polymers and the one or more nanoparticles are each comprised in the cross-linkable hydrogel composition in a concentration at which neither the one or more polymers nor the nanoparticles, taken alone, form a hydrogel, characterized in that the cross-linkable hydrogel precursor is not selectively adsorbed to the one or more nanoparticles and in that the cross-linkable hydrogel precursor and the one or more nanoparticles do not form a transient shear-thinning and self-healing hydrogel and further in that the cross-linkable hydrogel precursor is comprised in the cross-linkable hydrogel composition in a concentration at which the cross-linkable hydrogel precursor, taken alone, does not form a hydrogel.

In the context of the present invention, the term "gel" and "hydrogel" relates to materials in which the storage modulus (G') is greater than the loss modulus (G") at a frequency of 10 rad/s, as measured by oscillatory shear rheometry in the linear viscoelastic regime.

In a preferred embodiment of the cross-linkable hydrogel compositions according to the present invention, the one or more nanoparticles may comprise or consist of an inorganic or organic material. Exemplary materials are inorganic materials such as silica, titania, or organic materials such as synthetic polymers.

In a preferred embodiment of the cross-linkable hydrogel compositions according to the present invention, the one or more nanoparticles may comprise or consist of one or more block copolymers, in particular amphiphilic diblock copolymers, for example amphiphilic diblock copolymers having a polyether block such as PEG-b-PLA, PEG-*b*-PGA PEG-*b-*PCL, PEG-*b*-PLGA, PEG-*b*-PE PEG-*b*-PS where PEG is polyethylene glycol, PLA is polylactic acid, PGA is polyglycolic acid, PLGA is polylactic-co-polyglycolic acid, PCL is polycaprolactone, PE is polyethylene and PS is polystyrene. In an alternative embodiment, the one or more nanoparticles comprise, or consist of, PEGylated inorganic nanoparticles such as PEGylated iron oxide nanoparticles or PEGylated silica nanoparticles.

The one or more nanoparticles comprising or consisting of one or more amphiphilic diblock copolymers may be obtained by first dissolving the copolymers in a suitable organic solvent and then adding the solution dropwise into an aqueous or water phase under shear such as to precipitate the solved copolymer and form the nanoparticles.

In a preferred embodiment of the cross-linkable hydrogel compositions according to the present invention, the one or more nanoparticles may comprise or consist of PEG-*b*-PLA.

In a preferred embodiment of the cross-linkable hydrogel compositions according to the present invention, the cross-linkable hydrogel precursor and in particular in the case where the cross-linkable hydrogel precursor is a semisynthetic homo- or heteropolysaccharide, the polysaccharide may be substituted with acrylate or methacrylate moieties. Such a polysaccharide may be methacrylated hyaluronic acid or acrylated hyaluronic acid.

The degree of substitution of the polysaccharide substituted with acrylate or methacrylate moieties may be of from 0.1 to about 0.5, preferably of from 0.25 to 0.45 as determined by ¹H-NMR in D₂O from integration of the vinyl group normalized to the polysaccharide backbone. It has been found that too high a degree of substation of the polysaccharide negatively affect shelf life of the cross-linkable hydrogel compositions according to the present invention because of phase separation between the cross-linkable hydrogel precursor and the base carrier composition.

In a preferred embodiment of the present invention, the polyethylene glycol block in the diblock copolymer may be from 1 000 Da to 25 000 Da, preferably of from 1 000 Da to 5 000 Da and/or the polyhydroxy alkanoate block, and in particular the polylactic acid block, may be from 5 000 Da to 100 000 Da and preferably from 12 000 Da to 20 000 Da.

The shear-thinning and self-healing networks of the base carrier composition are formed by the appropriately paired nanoparticles and polymers, which selectively adsorb to the nanoparticles to form non-covalent cross-links that in turn lead to gel formation. Owing to the dynamic and transient nature of these cross-links, the base carrier composition exhibits dramatic shear-thinning and rapid self-healing.

The polymers and nanoparticles that form the shear-thinning and self-healing hydrogels of the base carrier composition each independently do not form a gel alone or are not used at a concentration where the polymer alone or nanoparticle alone form a gel. Only when the nanoparticle and polymer are combined does gel formation occur.

Thus, in the cross-linkable hydrogels according to the present invention, the loss modulus (G") for a solution of the polymer of the base carrier composition and the loss modulus (G") of a solution of the one or more nanoparticles of the base carrier composition are each greater than their respective storage moduli (G') at a frequency of 10 rad/s as measured by oscillatory shear rheometry in the linear viscoelastic regime; and wherein the one or more nanoparticles and the polymer of the base carrier composition form a hydrogel when combined. The storage modulus (G') for the resulting hydrogel is greater than the loss modulus (G") at a frequency of 10 rad/s as measured by oscillatory shear rheometry in the linear viscoelastic regime when combined.

In a preferred embodiment of the cross-linkable hydrogel compositions according to the present invention, the one or more nanoparticles have particle size Dₕ of between 20 nm and 100 nm, preferably between 45 nm and 65 or between 45 nm and 75 nm and/or the nanoparticles are present in an amount of between 1 and 25 wt% based on the weight of the cross-linkable hydrogel composition, and preferably between 10 and 20 wt% or between 5 and 20 wt% or between 2 and 20 wt%, based on the weight of the cross-linkable hydrogel composition. The Dₕ can be determined by dynamic light scattering at a scattering angle of 173° at 25 °C.

In a preferred embodiment of the cross-linkable hydrogel compositions according to the present invention, the cross-linkable hydrogel precursor is selected from the group consisting of synthetic or semisynthetic polymers, preferably bearing acrylate or methacrylate moieties, and more preferably having a degree of substitution of less than 0.5.

In a preferred embodiment of the cross-linkable hydrogel compositions according to the present invention, the one or more polymer of the aqueous base carrier composition is selected from the group consisting of semisynthetic polysaccharides, in particular from the group consisting of semisynthetic cellulose ethers or mixtures thereof such as methyl cellulose, ethyl cellulose, ethyl methyl cellulose, hydroxypropylmethyl cellulose, hydroxypropyl cellulose, hydroxyethyl cellulose and most preferably is hydroxypropylmethyl cellulose or hydroxyethyl cellulose, or mixtures thereof.

In a preferred embodiment of the cross-linkable hydrogel compositions according to the present invention, the one or more polymer of the aqueous base carrier composition, in particular in the case where the one or more polymer is a semisynthetic cellulose ether, has a molecular weight of from 100 kDa to 850 kDa, or from 10 kDa to 850 kDa, preferably of from 250 to 750 kDa, or from 50 kDa to 750 kDa.

In a preferred embodiment of the cross-linkable hydrogel compositions according to the present invention, the cross-linkable hydrogel composition further comprises one or more active pharmaceutical ingredients, in particular wherein the one or more active pharmaceutical ingredients are confined to the one or more nanoparticles of the base carrier composition. In the case where one or more active pharmaceutical ingredients are confined to the one or more nanoparticles, the one or more nanoparticles comprise, or consist of an amphiphilic diblock copolymer such as PEG-b-PLA, PEG-b-PGA PEG-b-PCL, PEG-b-PLGA, PEG-b-PE PEG-b-PS where PEG is polyethylene glycol, PLA is polylactic acid, PGA is polyglycolic acid, PLGA is polylactic-co-polyglycolic acid, PCL is polycaprolactone, PE is polyethylene and PS is polystyrene.

In a preferred embodiment of the cross-linkable hydrogel compositions according to the present invention, the cross-linkable hydrogel compositions further comprises one or more cells, preferably microorganism cells. The cells may be from yeasts, bacteria, plants or animals.

In a preferred embodiment of the cross-linkable hydrogel compositions according to the present invention, the cross-linkable hydrogel precursor is cross-linkable by radiation, change of pH, change of temperature or by contact with a crosslinking agent such as for example an ionic crosslinking agent.

The radiation wavelength needed for cross-linking the cross-linkable hydrogel compositions according to the present invention will depend on the type of cross-linkable hydrogel precursor used, and may for example be IR, visible or UV radiation. In the case where the cross-linkable hydrogel precursor is a synthetic or semisynthetic polymer bearing acrylate or methacrylate moieties, a suitable wavelength may be 405 nm.

The crosslinking agent needed for cross-linking the cross-linkable hydrogel compositions according to the present invention will depend on the type of cross-linkable hydrogel precursor used, and may for example be an ionic crosslinking agent such as metal ions. For instance, a suitable ionic crosslinking agent in the case where alginate is used as the cross-linkable hydrogel precursor may for example be divalent metal cations such as calcium or barium cations.

In a preferred embodiment of the cross-linkable hydrogel compositions according to the present invention, the cross-linkable hydrogel composition further comprises a photoinitiator capable of cross-linking the cross-linkable hydrogel precursor upon exposure to a cross-linking radiation, in particular when the cross-linkable hydrogel precursor is cross-linkable by radiation, such as when the cross-linkable hydrogel precursor is a synthetic or semisynthetic polymer bearing acrylate or methacrylate moieties, A suitable photoinitiator capable of cross-linking the cross-linkable hydrogel precursor upon exposure to a cross-linking radiation is lithium phenyl-2,4,6-trimethylbenzoylphosphinate (LAP), which may be comprised in the cross-linkable hydrogel composition according to the present invention in an amount of about 0.05 to 1 wt%, based on the weight of the cross-linkable hydrogel composition.

In a preferred embodiment of the cross-linkable hydrogel compositions according to the present invention, the cross-linkable hydrogel precursor is chosen from animal or plant homo- or heteropolysaccharides, such as alginate, and derivatives thereof, in particular methacrylated derivatives thereof such as methacrylated hyaluronic acid.

In a preferred embodiment of the cross-linkable hydrogel compositions according to the present invention, the cross-linkable hydrogel precursor is chosen from animal or plant homo- or heteropolysaccharides substituted with acrylate or methacrylate moieties, and which may have a degree of substitution of from 0.1 to about 0.5, preferably of from 0.25 to 0.45 as determined by 1H-NMR in D2O from integration of the vinyl group normalized to the polysaccharide backbone.

In a preferred embodiment of the cross-linkable hydrogel compositions according to the present invention, the cross-linkable hydrogel precursor is a protein such as collagen. Alternatively, the cross-linkable hydrogel precursor may be a protein such as laminin, fibronectin, or any natural or semisynthetic protein. Collagen can be cross-linked, as is known in the art, by controlling the pH such as to be between 6.5 to 8.5; preferably between 7.6 to 8.0 in an aqueous buffer of adjusted ionic strength. Suitable buffers can be based on PBS or HEPEs in addition to some salts such as for example NaCl.

In a preferred embodiment of the cross-linkable hydrogel compositions according to the present invention, the cross-linkable hydrogel precursor is a synthetic polymer, in particular a methacrylated or acrylated synthetic polymer such as a methacrylated or acrylated polypropylene glycol), poly(ethylene glycol) diacrylate or poly(ethylene propylene glycol), diacrylate.

It is further an object of the present invention to provide a use of a cross-linkable hydrogel composition according to the above as an ink for direct ink writing. The cross-linkable hydrogel composition exhibits shear-thinning and self-healing which allows for convenient extrusion through the nozzle of a direct ink writing apparatus and affords the self-support needed in a green state before cross-linking of the cross-linkable hydrogel precursor is induced, which cross-linking provides for stability in aqueous or biological environments.

It is further an object of the present invention to provide a use of a cross-linkable hydrogel composition according to the above as a vehicle for an injectable pharmaceutical depot formulation.

The cross-linkable hydrogel composition exhibits shear-thinning and self-healing which allows for convenient injection, for example via a syringe, into an injection site in a tissue before cross-linking of the cross-linkable hydrogel precursor is induced, which cross-linking provides for stability at the site of injection. In the case where the cross-linkable hydrogel composition includes an active pharmaceutical ingredient, the cross-linked cross-linkable hydrogel composition exhibits a controlled release of the active pharmaceutical ingredient, in particular when the active pharmaceutical ingredient is bound to the one or more nanoparticles. Because the nanoparticles are released more slowly from a depot of cross-linked cross-linkable hydrogel composition of the present invention when compared to a depot consisting of solely the base carrier composition, the release of the active pharmaceutical ingredient can be more accurately controlled.

It is further an object of the present invention to provide an object, obtained by cross-linking a green object of cross-linkable hydrogel composition according to the above.

The green object may be obtained in general by subjecting the cross-linkable hydrogel composition to shear such as to fluidize it and depositing the fluidized cross-linkable hydrogel composition, for example by direct ink writing the cross-linkable hydrogel composition or by injecting the cross-linkable hydrogel composition, prior to cross-linking the cross-linkable hydrogel composition.

It is further an object of the present invention to provide a process of manufacturing a object comprising the steps of
a. providing a cross-linkable hydrogel composition according to the above,
b. obtaining a green body comprising the cross-linkable hydrogel composition
c. cross-linking the green body to form the object, preferably photo-cross-linking the green body to form the object.

It is further an object of the present invention to provide a use for an aqueous base carrier composition in a DIW ink composition further comprising a cross-linkable hydrogel precursor, the aqueous base carrier composition comprising one or more hydrogel-forming polymers and one or more nanoparticles, wherein the one or more hydrogel-forming polymers are selectively adsorbed to the one or more nanoparticles, and wherein the one or more polymers and the one or more nanoparticles form a shear-thinning and self-healing hydrogel, and wherein the one or more hydrogel-forming polymers and the one or more nanoparticles are each comprised in the aqueous base carrier composition in a concentration at which neither the one or more polymers nor the nanoparticles, taken alone, form a hydrogel, characterized in that the cross-linkable hydrogel precursor is not selectively adsorbed to the one or more nanoparticles and in that the cross-linkable hydrogel precursor and the one or more nanoparticles do not form a shear-thinning and self-healing hydrogel and preferably in that the cross-linkable hydrogel precursor is comprised in the DIW ink composition in a concentration at which the cross-linkable hydrogel precursor, taken alone, does not form a hydrogel.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

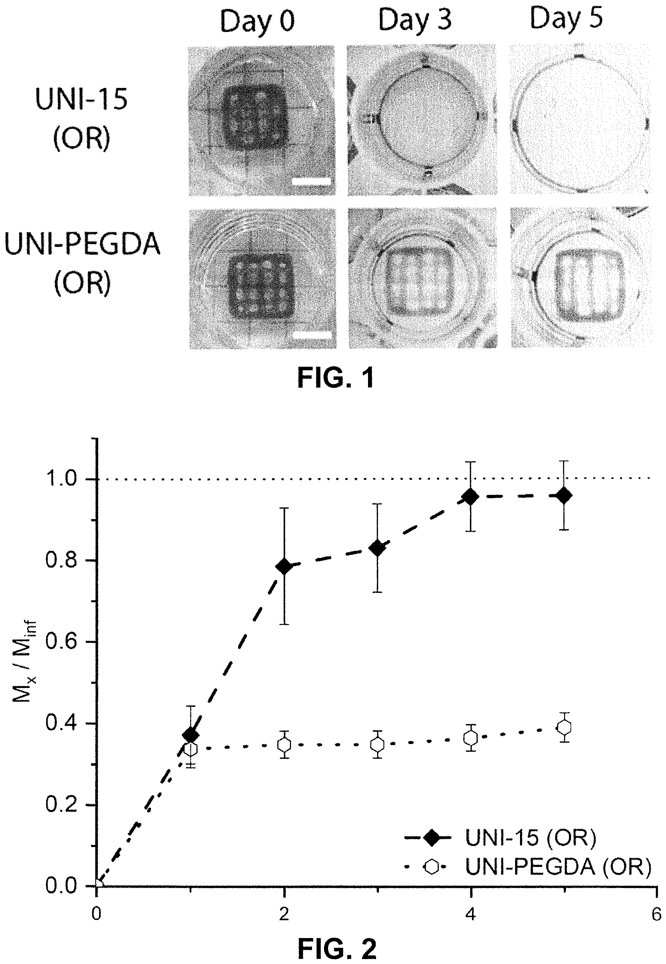

- Fig. 1: shows grid structures (8 × 8 mm) including a colorant bound to nanoparticles that were DIW printed using solely the aqueous base carrier composition, top row, and using the cross-linked cross-linkable hydrogel composition comprising both the aqueous base carrier composition and cross-linkable hydrogel precursor in the form of PEGDA (Example 4), bottom row.
- Fig. 2: shows the mass fraction of released colorant over a time of 5 days into an aqueous buffer from the grid structures of cross-linked cross-linkable hydrogel composition (Example 4), dotted line, and from the sole aqueous base carrier composition, dashed line.

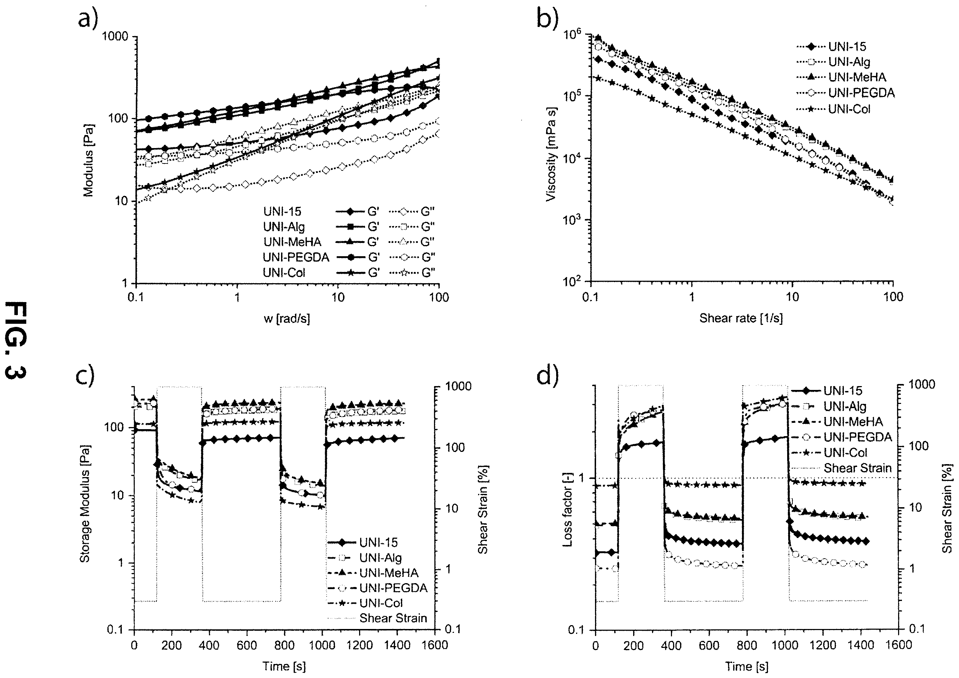

- Fig. 3: shows a) G' as well as G" over a frequency range (ω = 100 - 0.1 rad s⁻¹, γ = 0.3%, 25 °C) for compositions according to Examples 1 to 4 and for the sole aqueous base carrier composition, which was measured at 4 °C; b) Shear rate ramp (δy/δt = 0.1 - 100 s⁻¹) analysis for compositions according to Examples 1 to 4 and for the sole aqueous base carrier composition; c) Step strain analysis with alternating intervals at low (γ = 0.3%) and high (γ = 1000%) shear strain amplitude at constant angular frequency (ω = 10 rad s-1) for compositions according to Examples 1 to 4 and for the sole aqueous base carrier composition; d) self-healing was confirmed by plotting the loss factor (tan δ = G"/G') during the step strain analysis.

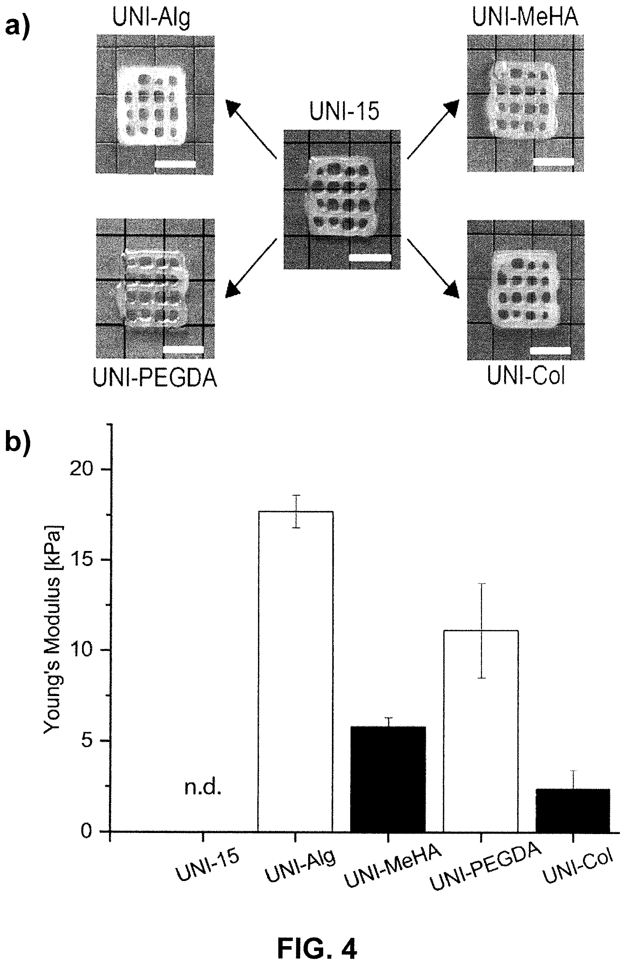

- Figure 4: shows a) grid structures (8 × 8 mm) that were DIW printed with the cross-linkable hydrogel compositions according to Examples 1-4 and solely base aqueous carrier composition, center, a straight needle was used at a constant velocity. 4b) indentation tests made on cross-linked cross-linkable hydrogel compositions according to Examples 1-4 and sole base carrier composition (UNI-15) after equilibration in PBS (pH 7.4, 37 °C). The indentation test could not be performed on sole base carrier composition (UNI-15).

### EXAMPLES

### Materials

Hydroxypropylmethylcellulose (HPMC; Mₙ ∼700 kDa, Ref. H3785), methacrylic anhydride (MA; Ref 276685), 4-(2-hydroxyethyl)-1-piperazine-ethanesulfonic acid (Hepes; 99%, Ref H3375), Oil Red O (OR; Ref O0625), calcium chloride (CaCl2; Ref 21074), anti-mouse IgG-Atto 594 (IgG-Atto 594; Ref 76085), 2,4,6-trimethylbenzoyl chloride (Ref 682519), dimethyl phenylphosphonite (Ref 149470), lithium bromide (Ref 213225), DAPI (Ref D9542) and 2-butanone (Ref 360473) were purchased from Sigma-Aldrich (Buchs, Switzerland). PEG5k-b-PLA16k (AK054) and PEGSk-b-PLA14k (AK049) were purchased from Akina (West Lafayette, IN, USA). Sodium alginate (Alg; Grade I-1G-80) was purchased from Kimica International (Tokyo, Japan); polyethylene glycol) diacrylate (PEGDA; Mw ~10 kDa, Ref ACRL-PEG-ACRL-10K) was purchased from Laysan Bio Inc. (Arab, AL, USA). Sodium hyaluronate (HA; Mw -700 kDa, Ref HA700K) was purchased from Lifecore (Chaska, MN, USA). Collagen (Col; 10 mg/ml, Ref 5133-20ML) was purchased from Advanced BioMatrix (San Diego, CA, USA). Acetone (Ref 20066.296) and acetonitrile (Ref 20071.294) were purchased from VWR Chemicals. Low glucose DMEM (Ref 22320-022), Pen Strep (PS, Ref 15140-122), FGF (Ref PHG0026), and fetal bovine serum (FBS; Ref 10270106), LIVE/DEAD Viability/Cytotoxicity kit (Ref L3224), AlexaFluor 488 Phalloidin (Ref A12379) were purchased from ThermoFisher.

### Preparation of PEG-b-PLA nanoparticles

The PEG-b-PLA nanoparticles were obtained by dissolving 140 mg PEG-b-PLA in 2 ml of acetone, from which solution the PEG-b-PLA nanoparticles were nanoprecipitated by dropwise addition (15 µL every 3 s) of said solution into 10 ml Milli-Q water under a high stir rate (650 rpm). After overnight evaporation of acetone, the thus obtained PEG-b-PLA nanoparticles were filtered by ultracentrifugation and re-suspended to a final concentration of 20 wt% in aqueous buffer (20 mM HEPES and 275 mM mannitol) or [for UNI, UNI-PEGDA, IINI-MeHA, and UNI-Alg] or to a final concentration of 25 wt% [ for UNI-Col)] in Milli-Q water.

The size of the PEG-b-PLA nanoparticles (Dₕ ∼55 nm) and dispersity of the PEG-b-PLA nanoparticles (PDI ~0.1) were characterized by dynamic light scattering on a Malvern ZetaSizer Nano ZS at a scattering angle of 173° and at 25 °C).

### Preparation of base carrier composition using PEG-b-PLA nanoparticles and HPMC (UNI-15)

An aqueous base carrier composition of HPMC and PEG-b-PLA nanoparticles was preprared by first dissolving HPMC in buffer solution (20 mM HEPES and 150 mM D-mannitol, pH = 7.4) at 1 wt% and allowed to equilibrate overnight (25 °C). Thereafter, the HPMC solution was combined with the 20 wt% nanoparticles in aqueous buffer (20 mM HEPES and 275 mM mannitol) to yield a base carrier composition of 15 wt% PEG-b-PLA nanoparticles and 1wt % HPMC.

### Example 1: Preparation of a cross-linkable hydrogel composition with PEG-b-PLA nanoparticles and HPMC as hydrogel-forming polymer with alginate as cross-linkable hydrogel precursor (UNI-Alg)

A composition was prepared by first dissolving 1 wt% HPMC in an aqueous buffer (20 mM HEPES and 150 mM D-mannitol, pH = 7.4) together with 1 wt% alginate and allowed to equilibrate overnight (25 °C). This composition comprising 1 wt% alginate and HPMC, respectively, was then combined and mixed with the above suspension of 20 wt% PEG-b-PLA nanoparticles in aqueous buffer (20 mM HEPES and 275 mM mannitol) such as to provide for the cross-linkable hydrogel composition according to the present invention comprising 1 wt% alginate, 15 wt% PEG-b-PLA nanoparticles and 1 wt% HPMC.

### Example 2: Preparation of of a cross-linkable hydrogel composition with PEG-b-PLA nanoparticles and HPMC as polymer with methacrylate hyaluronic acid as cross-linkable hydrogel precursor (UNI-MeHA).

An aqueous base carrier composition according to the present invention comprising 1 wt% methacrylate hyaluronic acid, 15 wt% PEG-b-PLA nanoparticles and 1 wt% HPMC was obtained in the same manner as above, with the exception that 1 wt% methacrylate hyaluronic acid was dissolved together with 1 wt% HPMC and 0.1 wt% lithium phenyl-2,4,6-trimethylbenzoylphosphinate (LAP) as photoinitiator in an aqueous buffer (20 mM HEPES and 150 mM D-mannitol, pH = 7.4).

The methacrylate hyaluronic had a degree of substitution of about 0.4.

### Example 3: Preparation of of a cross-linkable hydrogel composition with PEG-b-PLA nanoparticles and HPMC as polymer with polyethylene glycol) diacrylate (PEGDA) as cross-linkable hydrogel precursor(UNI-PEGDA)

An aqueous base carrier composition according to the present invention comprising 1 wt% polyethylene glycol) diacrylate, 15 wt% PEG-b-PLA nanoparticles and 5 wt% HPMC was obtained in the same manner as above, with the exception that 1 wt% polyethylene glycol) diacrylate was dissolved together with 5 wt% HPMC and 0.1 wt% lithium phenyl-2,4,6-trimethylbenzoylphosphinate (LAP) as photoinitiator in an aqueous buffer (20 mM HEPES and 150 mM D-mannitol, pH = 7.4).

### Example 4: Preparation of a cross-linkable hydrogel composition with PEG-b-PLA nanoparticles and HPMC as polymer with collagen as cross-linkable hydrogel precursor (UNI-Col)

A composition was prepared by first dissolving 1 wt% HPMC in Milli-Q water and allowed to equilibrate overnight (4 °C). This composition comprising 1 wt% HPMC was then combined and mixed with the above suspension of 25 wt% PEG-b-PLA nanoparticles in Milli-Q water such as to provide for the aqueous base carrier composition of the cross-linkable hydrogel composition according to the present invention comprising 1 wt% alginate, 15 wt% PEG-b-PLA nanoparticles and 1 wt% HPMC.

Collagen was_combined and mixed with the aqueous base carrier composition and until a concentration of 0.2 wt% collagen, 1 wt% HPMC and 15 wt% PEG-b-PLA nanoparticles reached.

### Example 5: Preparation of of a cross-linkable hydrogel composition with silica nanoparticles and HEC as polymer with methacrylate dextran as cross-linkable hydrogel precursor

A composition was prepared by first dissolving 1 wt% HEC in Milli-Q water and allowed to equilibrate overnight (4 °C). This composition comprising 1 wt% HEC was then combined and mixed a suspension of 25 wt% silica nanoparticles in Milli-Q water such as to provide for the aqueous base carrier composition of the cross-linkable hydrogel composition according to the present invention comprising 3 wt% methacrylate dextran, 15 wt% silica nanoparticles and 1 wt% HEC.

The methacrylate dextran had a degree of substitution of about 0.7.

While the composition exhibited desirable shear thinning and self-healing, the shelf life was limited in the sense that the composition would phase-separated within 24 hrs whereas with the other exemplary compositions no phase separation could be observed even after weeks or months.

### Cross-linking of the cross-linkable hydrogel compositions

The alginate cross-linkable hydrogel composition of Example 1 was cross-linked by immersing the scaffold in 100 mM CaCl₂ solution for 30 min.

The PEGDA and MeHA cross-linkable hydrogel compositions of Example 2 and 3 were cross-linked with visible light (λ = 405 nm, at I = 15 mW cm⁻²) for 5 min.

The collagen cross-linkable hydrogel composition of Example 4 was cross-linked by placing in an incubator (37 °C, 5% CO₂) for 120 min.

### Results

As can be seen from Figure 1, grid structures (8 × 8 mm) including a colorant bound to nanoparticles were DIW printed using solely aqueous base carrier composition as described above and using the cross-linked cross-linkable hydrogel composition comprising both the aqueous base carrier composition and cross-linkable hydrogel precursor in the form of PEGDA (Example 4). A rapid erosion was observed for the grid structures of sole aqueous base carrier composition, whereas for the grid structures of cross-linked cross-linkable hydrogel composition maintained its structure and released colorant over a longer time in the aqueous buffer.

As can be seen from Figure 2, the above grid structures of aqueous base carrier composition released the entire colorant over 5 days into an aqueous buffer whereas the grid structures of cross-linked cross-linkable hydrogel composition (Example 4) exhibited an initial burst release of ∼35% on day 1, followed by minimal release as the grid structures slowly eroded.

Thus, the cross-linkable hydrogel compositions according to the present invention exhibit extended stability and structural integrity in aqueous environments.

As can be seen from Figure 3 (3a/b/c/d), the cross-linkable hydrogel compositions according to the present invention of Examples 1-4 a) formed viscoelastic hydrogels, where G' > G" over the whole frequency range (ω = 100 - 0.1 rad s⁻¹, γ = 0.3%, 25 °C; the same behavior was observed for the aqueous base carrier composition, which was measured at 4 °C; b) Shear rate ramp (δy/δt = 0.1 - 100 s-1) analysis showed that shear-thinning behavior was maintained with the inclusion of each cross-linkable hydrogel precursor; c) Step strain analysis with alternating intervals at low (γ = 0.3%) and high (γ = 1000%) shear strain amplitude at constant angular frequency (ω = 10 rad s⁻¹) showed rapid and repeatable self-healing was maintained (~80% recovery in less than 60 sec) of each cross-linkable hydrogel precursor; d) self-healing was confirmed by plotting the loss factor (tan δ = G"/G') during the step strain analysis.

As can be seen from Figure 4a) grids (8 × 8 mm) were DIW printed with the cross-linkable hydrogel compositions according to the present invention of Examples 1-4 and solely base carrier composition. In all cases, a straight needle was used at a constant velocity. The pressure applied varied between ∼60 kPa for the sole base carrier composition and the cross-linkable hydrogel composition of Example 4, to -80 kPa for the cross-linkable hydrogel composition of Example 1, 2 and 3. The high printing fidelity illustrates the robustness of the cross-linkable hydrogel compositions; 4b) indentation tests on cross-linked cross-linkable hydrogel compositions of Examples 1-4 and sole base carrier composition (UNI) after equilibration in PBS (pH 7.4, 37 °C). The indentation test could not be performed on sole base carrier composition (UNI).

## Claims

1. A cross-linkable hydrogel composition comprising an aqueous base carrier composition and a cross-linkable hydrogel precursor, wherein the aqueous base carrier composition comprises one or more polymers and one or more nanoparticles, wherein the one or more polymers are selectively adsorbed to the one or more nanoparticles, and wherein the one or more polymers and the one or more nanoparticles form a shear-thinning and self-healing hydrogel and wherein the one or more polymers and the one or more nanoparticles are each comprised in the cross-linkable hydrogel composition in a concentration at which neither the one or more polymers nor the nanoparticles, taken alone, form a hydrogel, **characterized in that** the cross-linkable hydrogel precursor is not selectively adsorbed to the one or more nanoparticles and **in that** the cross-linkable hydrogel precursor and the one or more nanoparticles do not form a shear-thinning and self-healing hydrogel and further **in that** the cross-linkable hydrogel precursor is comprised in the cross-linkable hydrogel composition in a concentration at which the cross-linkable hydrogel precursor, taken alone, does not form a hydrogel.

2. The cross-linkable hydrogel composition according to claim 1, wherein the one or more nanoparticles have particle size Dₕ of between 20 nm and 100 nm, preferably between 45 nm and 65 and/or the nanoparticles are present in an amount of between 1 and 25 wt% based on the weight of the cross-linkable hydrogel composition, and preferably between 10 and 20 wt% based on the weight of the cross-linkable hydrogel composition.

3. The cross-linkable hydrogel composition according to claim 1 or 2, wherein the one or more polymer of the aqueous base carrier composition is selected from the group consisting of semisynthetic polysaccharides, in particular from the group consisting of semisynthetic cellulose ethers or mixtures thereof such as methyl cellulose, ethyl cellulose, ethyl methyl cellulose, hydroxypropylmethyl cellulose, hydroxypropyl cellulose, hydroxyethyl cellulose and most preferably is hydroxypropylmethyl cellulose or hydroxyethyl cellulose, or mixtures thereof.

4. The cross-linkable hydrogel composition according to any preceding claim, wherein the one or more polymer of the aqueous base carrier composition has a molecular weight of from 100 kDa to 850 kDa.

5. The cross-linkable hydrogel composition according to any preceding claim , wherein it further comprises one or more active pharmaceutical ingredients, in particular wherein the one or more active pharmaceutical ingredients are confined to the one or more nanoparticles of the base carrier composition.

6. The cross-linkable hydrogel composition according to any preceding claim , wherein it further comprises one or more cells, preferably microorganism cells.

7. The cross-linkable hydrogel composition according to any preceding claim , wherein the cross-linkable hydrogel precursor is cross-linkable by radiation, change of pH, change of temperature or by contact with a crosslinking agent such as for example an ionic crosslinking agent.

8. The cross-linkable hydrogel composition according to any preceding claim , wherein the cross-linkable hydrogel composition further comprises a photoinitiator capable of cross-linking the cross-linkable hydrogel precursor upon exposure to a cross-linking radiation.

9. The cross-linkable hydrogel composition according to any preceding claim , wherein the cross-linkable hydrogel precursor is chosen from animal or plant homo- or heteropolysaccharides, such as alginate, and methacrylates or acrylates thereof, such as methacrylated hyaluronic acid.

10. The cross-linkable hydrogel composition according to any preceding claim, wherein the cross-linkable hydrogel precursor is a protein such as collagen.

11. The cross-linkable hydrogel composition according to any preceding claim, wherein the cross-linkable hydrogel precursor is a synthetic polymer, in particular an acrylated or methacrylated synthetic polymer a such as polyethylene glycol) diacrylate.

12. Use of a cross-linkable hydrogel composition according to any preceding claims as an ink for direct ink writing.

13. Use of a cross-linkable hydrogel composition according to any of claims 1 to 11 as a vehicle for an injectable pharmaceutical formulation.

14. An object obtained by cross-linking a green object of cross-linkable hydrogel composition according to claim 1 to 11 or comprising the cross-linked cross-linkable hydrogel composition according to claim 1 to 11.

15. A process of manufacturing an object comprising the steps of
a. providing a cross-linkable hydrogel composition according to claim 1 to 11,
b. obtaining a green body comprising the cross-linkable hydrogel composition
c. cross-linking the green body to form the object or a part thereof, preferably photo-cross-linking the green body to form the object or a part thereof.

## Patentansprüche

1. Eine vernetzbare Hydrogel-Zusammensetzung, aufweisend eine Trägerzusammensetzung mit wässriger Basis und einen vernetzbaren Hydrogel-Vorläufer; wobei die Trägerzusammensetzung mit wässriger Basis ein Polymer oder mehrere Polymere aufweist, und einen oder mehrere Nanopartikel, wobei das eine Polymer oder die mehreren Polymere selektiv an dem einen Nanopartikel oder den mehreren Nanopartikeln adsorbiert werden, und wobei das eine Polymer oder die mehreren Polymere und der eine Nanopartikel oder die mehreren Nanopartikel ein Hydrogel mit Strukturviskosität und Selbstheilungsfunktion bilden und wobei das eine Polymer oder die mehreren Polymere und der eine Nanopartikel oder die mehreren Nanopartikel jeweils in der vernetzbaren Hydrogel-Zusammensetzung in einer Konzentration enthalten sind, bei welcher weder das eine Polymer oder die mehreren Polymere noch die Nanopartikel, für sich alleine ein Hydrogel bilden, **dadurch gekennzeichnet, dass** der vernetzbare Hydrogel-Vorläufer nicht selektiv an dem einen Nanopartikel oder den mehreren Nanopartikeln adsorbiert wird und dass der vernetzbare Hydrogel-Vorläufer und der eine Nanopartikel oder die mehreren Nanopartikel kein Hydrogel mit Strukturviskosität und Selbstheilungsfunktion bilden und dass ferner der vernetzbare Hydrogel-Vorläufer in der vernetzbaren Hydrogel-Zusammensetzung in einer Konzentration enthalten ist, bei welcher der vernetzbare Hydrogel-Vorläufer für sich alleine kein Hydrogel bildet.

2. Vernetzbare Hydrogel-Zusammensetzung gemäss Anspruch 1, wobei der eine Nanopartikel oder die mehreren Nanopartikel eine Partikelgrösse Dₕ von zwischen 20 nm und 100 nm aufweisen, vorzugsweise zwischen 45 nm und 65 nm und/oder wobei die Nanopartikel in einer Menge von zwischen 1 und 25 Gewichtsprozent vorliegen, basierend auf dem Gewicht der vernetzbaren Hydrogel-Zusammensetzung, und vorzugsweise zwischen 10 und 20 Gewichtsprozent, basierend auf dem Gewicht der vernetzbaren Hydrogel-Zusammensetzung.

3. Vernetzbare Hydrogel-Zusammensetzung gemäss Anspruch 1 oder 2, wobei das eine Polymer oder die mehreren Polymere der Trägerzusammensetzung mit wässriger Basis ausgewählt ist bzw. ausgewählt sind aus der Gruppe bestehend aus halbsynthetischen Polysacchariden, insbesondere aus der Gruppe bestehend aus halbsynthetischen Zelluloseethern oder Mischungen davon, wie beispielsweise Methylzellulose, Ethylzellulose, Ethylmethylzellulose, Hydroxypropylmethylzellulose, Hydroxypropylzellulose, Hydroxyethylzellulose, wobei es sich am meisten bevorzugt um Hydroxypropylmethylzellulose oder Hydroxyethylzellulose oder Mischungen davon handelt.

4. Vernetzbare Hydrogel-Zusammensetzung gemäss einem der vorhergehenden Ansprüche, wobei das eine Polymer oder die mehreren Polymere der Trägerzusammensetzung mit wässriger Basis ein Molekulargewicht von 100kDa bis 850 kDa aufweist bzw. aufweisen.

5. Vernetzbare Hydrogel-Zusammensetzung gemäss einem der vorhergehenden Ansprüche, ferner aufweisend einen aktiven pharmazeutischen Inhaltsstoff oder mehrere aktive pharmazeutische Inhaltsstoffe, wobei insbesondere der eine aktive pharmazeutische Inhaltsstoff oder die mehreren aktiven pharmazeutischen Inhaltsstoffe begrenzt sind auf den einen Nanopartikel oder die mehreren Nanopartikel der Basis-Trägerzusammensetzung.

6. Vernetzbare Hydrogel-Zusammensetzung gemäss einem der vorhergehenden Ansprüche, ferner aufweisend eine Zelle oder mehrere Zellen, vorzugsweise Mikroorganismus-Zellen.

7. Vernetzbare Hydrogel-Zusammensetzung gemäss einem der vorhergehenden Ansprüche, wobei der vernetzbare Hydrogel-Vorläufer vernetzbar ist durch Strahlung, pH-Änderung, Temperaturänderung, oder durch Kontakt mit einem Vernetzungsmittel, wie beispielsweise ein ionisches Vernetzungsmittel.

8. Vernetzbare Hydrogel-Zusammensetzung gemäss einem der vorhergehenden Ansprüche, wobei die vernetzbare Hydrogel-Zusammensetzung ferner einen Photoinitiator aufweist, welcher fähig ist, den vernetzbaren Hydrogel-Vorläufer zu vernetzen indem dieser einer vernetzenden Strahlung ausgesetzt wird.

9. Vernetzbare Hydrogel-Zusammensetzung gemäss einem der vorhergehenden Ansprüche, wobei der vernetzbare Hydrogel-Vorläufer ausgewählt ist aus tierischen oder pflanzlichen Homo- oder Heteropolysacchariden, wie beispielsweise Alginat, und Methacrylaten oder Acrylaten davon, wie beispielsweise methacrylierte Hyaluronsäure.

10. Vernetzbare Hydrogel-Zusammensetzung gemäss einem der vorhergehenden Ansprüche, wobei der vernetzbare Hydrogel-Vorläufer ein Protein ist, wie beispielsweise ein Kollagen.

11. Vernetzbare Hydrogel-Zusammensetzung gemäss einem der vorhergehenden Ansprüche, wobei der vernetzbare Hydrogel-Vorläufer ein synthetisches Polymer ist, insbesondere ein acryliertes oder methacryliertes synthetisches Polymer, wie beispielsweise ein Poly(ethylenglykol)diacrylat.

12. Verwendung einer vernetzbaren Hydrogel-Zusammensetzung gemäss einem der vorhergehenden Ansprüche als eine Tinte für Direct-Ink-Writing.

13. Verwendung einer vernetzbaren Hydrogel-Zusammensetzung gemäss einem der Ansprüche 1 bis 11 als ein Trägerstoff für eine injizierbare pharmazeutische Formulierung.

14. Gegenstand, welcher durch Vernetzen eines unvernetzten Gegenstands einer vernetzbaren Hydrogel-Zusammensetzung gemäss Anspruch 1 bis 11 gewonnen wird, oder welcher die vernetzte vernetzbare Hydrogel-Zusammensetzung gemäss Anspruch 1 bis 11 aufweist.

15. Verfahren zur Herstellung eines Gegenstands, beinhaltend die folgenden Schritte:
a. Bereitstellen einer vernetzbaren Hydrogel-Zusammensetzung gemäss Anspruch 1 bis 11,
b. Gewinnen eines unvernetzten Körpers, aufweisend die vernetzbare Hydrogel-Zusammensetzung,
c. Vernetzen des unvernetzten Körpers zur Bildung des Gegenstands oder eines Teils davon, vorzugsweise Photovernetzen des unvernetzten Körpers zur Bildung des Gegenstands oder eines Teils davon.

## Revendications

1. Une composition d'hydrogel réticulable comprenant une composition de support de base aqueuse et un précurseur d'hydrogel réticulable, dans laquelle la composition de support de base aqueuse comprend un ou plusieurs polymères et une ou plusieurs nanoparticules, dans laquelle l'un ou plusieurs polymères sont sélectivement adsorbés sur l'une ou plusieurs nanoparticules, et dans laquelle l'un ou plusieurs polymères et l'une ou plusieurs nanoparticules forment un hydrogel rhéofluidifiant et auto-cicatrisant et dans laquelle l'un ou plusieurs polymères et l'une ou plusieurs nanoparticules sont chacun/chacune compris dans la composition d'hydrogel réticulable dans une concentration à laquelle ni l'un ou plusieurs polymères ni l'un ou les nanoparticules, individuellement, ne forment un hydrogel, **caractérisée en ce que** le précurseur d'hydrogel réticulable n'est pas sélectivement adsorbé sur l'une ou plusieurs nanoparticules et **en ce que** le précurseur d'hydrogel réticulable et l'une ou plusieurs nanoparticules ne forment pas un hydrogel rhéofluidifiant et autocicatrisant et en outre **en ce que** le précurseur d'hydrogel réticulable est compris dans la composition d'hydrogel réticulable à une concentration à laquelle le précurseur d'hydrogel réticulable, individuellement, ne forme pas d'hydrogel.

2. La composition d'hydrogel réticulable selon la revendication 1, dans laquelle l'un ou plusieurs nanoparticules ont une taille de particule Dₕ comprise entre 20 nm et 100 nm, de préférence entre 45 nm et 65 nm et/ou les nanoparticules sont présentes en une quantité comprise entre 1 et 25 % en poids sur la base du poids de la composition d'hydrogel réticulable, et de préférence entre 10 et 20 % en poids sur la base du poids de la composition d'hydrogel réticulable.

3. La composition d'hydrogel réticulable selon la revendication 1 ou 2, dans laquelle l'un ou plusieurs polymères de la composition de support de base aqueuse sont choisis dans le groupe constitué de polysaccharides semi-synthétiques, en particulier dans le groupe constitué d'éthers de cellulose semi-synthétiques ou de mélanges de ceux-ci, tels que la méthylcellulose, l'éthylcellulose, l'éthylméthylcellulose, l'hydroxypropylméthylcellulose, l'hydroxypropylcellulose, l'hydroxyéthylcellulose et de préférence l'hydroxypropylméthylcellulose ou l'hydroxyéthylcellulose, ou leurs mélanges.

4. La composition d'hydrogel réticulable selon l'une quelconque des revendications précédentes, dans laquelle l'un ou plusieurs polymères de la composition de support de base aqueuse ont un poids moléculaire de 100 kDa à 850 kDa.

5. La composition d'hydrogel réticulable selon l'une quelconque des revendications précédentes, dans laquelle elle comprend en outre un ou plusieurs ingrédients pharmaceutiques actifs, en particulier dans laquelle l'un ou plusieurs ingrédients pharmaceutiques actifs sont confinés à l'une ou plusieurs nanoparticules de la composition de support de base.

6. La composition d'hydrogel réticulable selon l'une quelconque des revendications précédentes, dans laquelle elle comprend en outre une ou plusieurs cellules, de préférence des cellules de microorganisme.

7. La composition d'hydrogel réticulable selon l'une quelconque des revendications précédentes, dans laquelle le précurseur d'hydrogel réticulable est réticulable par changement de pH, par irradiation, par changement de température ou par contact avec un agent de réticulation tel que, par exemple, un agent de réticulation ionique.

8. La composition d'hydrogel réticulable selon l'une quelconque des revendications précédentes, dans laquelle la composition d'hydrogel réticulable comprend en outre un photoinitiateur capable de réticuler le précurseur d'hydrogel réticulable lors de l'exposition à un rayonnement de réticulation.

9. La composition d'hydrogel réticulable selon l'une quelconque des revendications précédentes, dans laquelle le précurseur d'hydrogel réticulable est choisi parmi les homo- ou hétéropolysaccharides animaux ou végétaux, tels que l'alginate, et leurs dérivés, notamment leurs dérivés méthacrylés ou acrylés, tel que l'acide hyaluronique méthacrylé.

10. La composition d'hydrogel réticulable selon l'une quelconque des revendications précédentes, dans laquelle le précurseur d'hydrogel réticulable est une protéine, telle que le collagène.

11. La composition d'hydrogel réticulable selon l'une quelconque des revendications précédentes, dans laquelle le précurseur d'hydrogel réticulable est un polymère synthétique, en particulier un polymère synthétique acrylé ou méthacrylé, tel que le diacrylate de polyéthylène glycol.

12. Utilisation d'une composition d'hydrogel réticulable selon l'une quelconque des revendications précédentes comme encre pour l'écriture directe à l'encre.

13. Utilisation d'une composition d'hydrogel réticulable selon l'une quelconque des revendications 1 à 11, comme véhicule pour une formulation pharmaceutique injectable.

14. Un objet obtenu par réticulation d'un objet cru en composition d'hydrogel réticulable selon les revendications 1 à 11 ou comprenant la composition d'hydrogel réticulable réticulée selon les revendications 1 à 11.

15. Le procédé de fabrication d'un objet comprenant les étapes de
a) fournir une composition d'hydrogel réticulable selon la revendication 1 à 11,
b) obtenir un corps cru comprenant la composition d'hydrogel réticulable,
c) réticuler le corps cru pour former l'objet ou une partie de celui-ci, de préférence photo-réticuler le corps cru pour former l'objet ou une partie de celui-ci.
